# EUROPEAN PATENT APPLICATION

(11) **EP 4 252 636 A1**
(43) Date of publication of application: **04.10.2023**
(21) Application number: 22165791.9
(22) Date of filing: 31.03.2022
(51) Int. Cl.: A61B 5/026, A61B 6/00, A61B 5/0295, A61B 5/00

(54) **PERFUSION MONITORING**

(71) Applicant: Koninklijke Philips N.V., 5656 AG Eindhoven (NL)
(72) Inventor: WISSEL, Tobias, Eindhoven (NL); KRÖNKE, Sven, Eindhoven (NL); LUCASSEN, Gerhardus Wilhelmus, Eindhoven (NL); VERHAGEN, Rieko, Eindhoven (NL); NOTTEN, Marc, Eindhoven (NL)
(74) Representative: Philips Intellectual Property & Standards

(57) **Abstract**

The present invention relates to perfusion monitoring. In order to provide facilitated peripheral perfusion monitoring, a device (10) for monitoring peripheral perfusion is provided that comprises a data input (12), a data processor (14) and an output interface (16). The data input is configured to provide angiographic image data comprising information about macrovascular blood flow in an area of interest of a subject. The angiographic image data comprises first image data relating to a first point in time, and second image data relating to a second point in time. The data processor is configured to compare the first image data and the second image data to identify, within the area of interest of the subject, a vascular region of interest for macrovascular flow. The data processor is also configured to determine at least one tissue portion of interest for microvascular perfusion based on the identified vascular region of interest and based on feeding information assigned to the identified vascular region of interest. The data processor is further configured to determine a surface portion for assessing microvascular perfusion in the at least one tissue portion of interest with optical perfusion imaging. The data processor is furthermore configured to allocate the surface portion on an outer surface of the subject. The output interface is configured to provide a surface portion identifier based on the allocated surface portion.

## Description

### FIELD OF THE INVENTION

The present invention relates to perfusion monitoring and relates in particular to a device for monitoring peripheral perfusion, to a system for monitoring peripheral perfusion and to a method for peripheral perfusion imaging.

### BACKGROUND OF THE INVENTION

Atherosclerosis in the peripheral arteries is a chronic and slowly developing condition causing narrowing of the arteries. Depending on the degree of narrowing, various symptoms may occur with patients developing acute events associated with thrombosis and/or embolism and occlusion of a major artery. Narrowing of the arteries leads to peripheral artery disease (PAD). Narrowing of arteries can also result in coronary artery disease. Current clinical methods to diagnose PAD are based on blood pressure measurements like Ankle Brachial Index (ABI), Toe Brachial Index (TBI) and Toe Pressure (TP), which measure ratios of blood pressures at ankle or toe and the arm. Further, transcutaneous oxygen tension (TcpO2) measurements are performed to get oxygen tension values after heating tissue, but these measurements are cumbersome. Furthermore, to look at the global perfusion, digital subtraction angiography (DSA) is used which provides a 2D projection of the vascular system where injected contrast fluid flows through the artery and veins. Stenoses due to calcified arteries or compressed veins, thrombosis or a lack of perfusion at the lower limbs as their consequence can be readily visualized, but sometimes at the cost of radiation and contrast. EP 3808275 A1 provides perfusion angiography combined with photoplethysmography imaging for peripheral vascular disease assessment. However, concurrent acquisition provides a complex workflow.

### SUMMARY OF THE INVENTION

There may thus be a need to provide facilitated peripheral perfusion monitoring.

The object of the present invention is solved by the subject-matter of the independent claims; further embodiments are incorporated in the dependent claims. It should be noted that the following described aspects of the invention apply also for the device for monitoring peripheral perfusion, for the system for monitoring peripheral perfusion and for the method for peripheral perfusion imaging.

According to the present invention, a device for monitoring peripheral perfusion is provided. The device comprises a data input, a data processor and an output interface. The data input is configured to provide angiographic image data comprising information about macrovascular blood flow in an area of interest of a subject. The angiographic image data comprises first image data relating to a first point in time, and second image data relating to a second point in time. The data processor is configured to compare the first image data and the second image data to identify, within the area of interest of the subject, a vascular region of interest for macrovascular flow. The data processor is also configured to determine at least one tissue portion of interest for microvascular perfusion based on the identified vascular region of interest and based on feeding information assigned to the identified vascular region of interest. The data processor is further configured to determine a surface portion for assessing microvascular perfusion in the at least one tissue portion of interest with optical perfusion imaging. The data processor is furthermore configured to allocate the surface portion on an outer surface of the subject. The output interface is configured to provide a surface portion identifier based on the allocated surface portion.

As an effect, the present invention enables to directly link re-opened macrovasculature as an immediate effect of the intervention to the optical measures of the actual perfusion at the symptomatic site. Angiography as basis thus provides an improved guidance of optical measurements and a confirmation of specific angiographic observations at the skin surface.

According to an example, the data processor is configured to generate a perfusion hypothesis based on the comparing of the first image data and the second image data and the identification of the vascular region of interest for macrovascular flow. The data processor is configured to generate, as the identifier, instructions for optical perfusion imaging for at least one of the group of quantifying and validating of the perfusion hypothesis.

According to an example, the data input is configured to provide at least one of the group of locational information and semantic information for the vascular structure of at least one of the group of the first image data and the second image data. The data processor is configured to determine the at least one tissue portion of interest for microvascular perfusion using the semantic information. In addition or alternatively, the data processor is configured to allocate the surface portion using the locational information.

According to an example, the angiographic image data comprises spatial anatomical information comprising a plurality of vessel segments. For the identification of the vascular region of interest for macrovascular flow, the data processor is configured to identify at least one of the plurality of vessel segments. The feeding information comprises segments of tissue portions that are assigned to vessel segments of the plurality of vessel segments as being supplied by these vessel segments.

According to an example, the feeding information comprises a feeding territory model. The data processor is configured to spatially register the angiographic image data to the feeding territory model. The data processor is configured to register the feeding territory model to the subject.

According to an example, the output interface is configured to provide optical perfusion image data. The optical perfusion image data comprises at least one 2D optical perfusion image of the surface of the subject. The data processor is configured to generate an indicator for the allocated surface portion. The data processor is also configured to overlay the indicator to the at least one 2D optical perfusion image to provide a location augmented optical perfusion image. The output interface is configured to provide the location augmented optical perfusion image for a perfusion assessment in the image portion defined by the indicator.

According to the present invention, also a system for monitoring peripheral perfusion is provided. The system comprises an X-ray imaging arrangement for angiography of a region of interest of a subject, an optical perfusion arrangement for measurement of perfusion within a surface of the subject and a device for monitoring peripheral perfusion according to one of the preceding examples. The X-ray imaging system is configured to provide the angiographic image data. The optical perfusion arrangement is configured to conduct optical perfusion imaging of the allocated surface portion based on the surface portion identifier.

According to an example, a spatial correspondence arrangement is provided that is configured to provide relative spatial correspondence data of the X-ray imaging arrangement and the optical perfusion arrangement.

In an example, a location detection system is provided to detect current positions and locations of both arrangements. For example, the location detection is based on electromagnetic markers. In another example, optical detection of marker locations is provided.

According to the present invention, also a method for peripheral perfusion imaging is provided. The method comprises the following steps:
- Providing angiographic image data comprising information about macrovascular blood flow in an area of interest of a subject; the angiographic image data comprises first image data relating to a first point in time and second image data relating to a second point in time.
- Comparing the first image data and the second image data to identify, within the area of interest of the subject, a vascular region of interest for macrovascular flow.
- Determining at least one tissue portion of interest for microvascular perfusion based on the identified vascular region of interest and based on feeding information assigned to the identified vascular region of interest.
- Determining a surface portion for assessing microvascular perfusion in the at least one tissue portion of interest with optical perfusion imaging.
- Allocating the surface portion on an outer surface of the subject; and
- Providing a surface portion identifier based on the allocated surface portion.

According to an aspect, the invention proposes to locate and spatially link a (macrovascular) perfusion signal of an angiography (or lack thereof) to a relevant surface area for (i) choosing the most relevant field-of-view for an optical measurement, and (ii) for validating a re-perfusion hypothesis as expected from the perfusion angiography against data from the optical surface perfusion map.

According to an aspect, two modalities are combined in a stereo-imaging configuration for transferring information from angiographic to optical surface measurements for obtaining sophisticated insights into perfusion properties of limbs. A macrovascular perfusion signal of the angiography (or a lack thereof) is located and spatially linked to the relevant surface area to (i) choose the most relevant field-of-view for the optical measurement, and, as an option, (ii) to validate re-perfusion hypotheses as expected from perfusion angiography against data from the optical surface perfusion map. As an example, optical perfusion monitoring such as iPPG, MSI, SLI or combinations thereof measure the blood content, flow or oxygenation directly where necrotic wounds and other symptoms actually occur - within a few millimeters of the skin surface.

In an example, it is provided to measure peripheral perfusion and/or tissue oxygenation at the skin surface using optical illumination with e.g. contact probes, such as laser doppler imaging, speckle contrast imaging, or with non-contact imaging methods using a camera, e.g. imaging photoplethysmography (iPPG), laser speckle imaging (LSI) or multi/hyperspectral imaging (MSI/HSI). These techniques give insight of the microvascular perfusion and oxygenation which can then be provided to decide on e.g. treatment, predict wound healing and outcome.

In an example, a system comprises an optical perfusion measurement device, e.g. hyper- or multispectral iPPG with structured light, and a post-processing unit or module that maps the measured signal, e.g. diffuse reflectance spectrum, to hemodynamic parameters, e.g. oxygen saturation. The system further comprises an angiography imaging system, e.g. a C-arm X-ray imaging system, that may be provided in a stereo configuration with respect to an optical imaging system, with known or measurable relative positioning/orientation, e.g. by using senor hardware or by pose-estimation using the acquired images. The system furthermore comprises an analysis unit or module with a user interface, which establishes spatial correspondence between the modalities and transfers locational information and also hypotheses about the perfusion status from angiography to the optical surface domain for an advanced analysis of perfusion properties. The present invention leverages the complementary properties of optical and angiographic perfusion measurements and overcomes up to a certain degree the limitations of one modality by using the other modality.

As an example, when referring to a combination of photoplethysmogram imaging (iPPG), multi-spectral imaging (MSI) and structured light imaging (SLI), chemical sensitivity (oxygenation etc.) is provided. A penetration depth of approximately 2mm is possible, which has to be seen though in addition to a depth of larger than 10 cm of the angiography imaging. Further, a typical view of imaging may be provided as sole/en-face, i.e. surface imaging, in addition to angiography's usually lateral volume projection. The optical imaging is providing a high sensitivity to microvascular perfusion, whereas angiography is providing a high sensitivity to macrovascular perfusion. The optical imaging enables to observe blood perfusion related parameters in absolute units, such as for oxygen saturation, or SLI can facilitate blood-volume-content measurements. Since optical imaging is a purely optical measurement, the subject is not exposed to any additional radiation dose. As a further complementation, optical imaging may be provided with a frame rate of approximately 24 frames per second, in addition to a frame rate of e.g. three frames per second of angiography imaging.

As an aspect, both imaging modalities provide relevant and complementary pieces of information about the effect of an intervention on the perfusion status. The present invention provides the explicit connection of the information.

As an example, DSA sequences are acquired, e.g. in a repetitive manner, to evaluate re-perfusion in the macrovascular tree in the foot. Seeing increases in perfusion in previously ischemic regions gives rise to the hypothesis that tissue perfusion and oxygenation in the surface wound area are also positively affected. This hypothesis is then quantified and used for a tailored optical validation. In an example, the present invention identifies perfusion deficits and regions of interest in the angiography and uses this for guiding the optical measurement. Explicit hypotheses on how an angiographically observed change in perfusion will affect tissue perfusion at the skin surface, are formulated and brought into the optical measurement for validation.

These and other aspects of the present invention will become apparent from and be elucidated with reference to the embodiments described hereinafter.

### BRIEF DESCRIPTION OF THE DRAWINGS

Exemplary embodiments of the invention will be described in the following with reference to the following drawings:
Fig. 1 schematically shows an example of a device for monitoring peripheral perfusion.
Fig. 2 shows an example of a system for monitoring peripheral perfusion comprising an example of the device for monitoring peripheral perfusion of Fig. 1.
Fig. 3 shows steps of an example of a method for peripheral perfusion imaging.
Fig. 4 illustrates an example of a workflow for peripheral perfusion imaging.
Fig. 5 illustrates a further example of a workflow for peripheral perfusion imaging.

### DETAILED DESCRIPTION OF EMBODIMENTS

Certain embodiments will now be described in greater details with reference to the accompanying drawings. In the following description, like drawing reference numerals are used for like elements, even in different drawings. The matters defined in the description, such as detailed construction and elements, are provided to assist in a comprehensive understanding of the exemplary embodiments. Also, well-known functions or constructions are not described in detail since they would obscure the embodiments with unnecessary detail. Moreover, expressions such as "at least one of', when preceding a list of elements, modify the entire list of elements and do not modify the individual elements of the list.

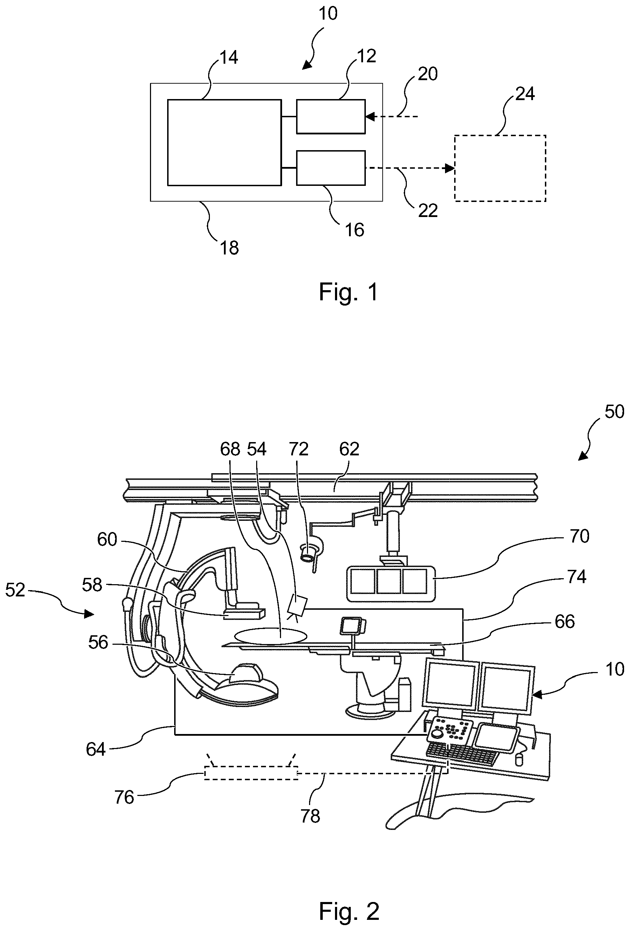

Fig. 1 schematically shows an example of a device 10 for monitoring peripheral perfusion. The device 10 comprises a data input 12, a data processor 14 and an output interface 16.

The data input 12 is configured to provide angiographic image data comprising information about macrovascular blood flow in an area of interest of a subject. The angiographic image data comprises first image data relating to a first point in time, and second image data relating to a second point in time.

The data processor 14 is configured to compare the first image data and the second image data to identify, within the area of interest of the subject, a vascular region of interest for macrovascular flow. The data processor 14 is also configured to determine at least one tissue portion of interest for microvascular perfusion based on the identified vascular region of interest and based on feeding information assigned to the identified vascular region of interest. The data processor 14 is further configured to determine a surface portion for assessing microvascular perfusion in the at least one tissue portion of interest with optical perfusion imaging. The data processor 14 is furthermore configured to allocate the surface portion on an outer surface of the subject.

The output interface 16 is configured to provide a surface portion identifier based on the allocated surface portion.

The data input 12, the data processor 14 and the output interface 16 can be provided in an integrated manner, as indicated by a first frame 18. In another option, the data input 12, the data processor 14 and the output interface 16 are provided as separate arrangements.

A first hashed arrow 20 indicates the provision of the above mentioned supply data, and a second hashed arrow 22 indicates the generated data e.g. in form of the surface portion identifier.

The surface portion identifier may be provided on a display 24 indicated with a second frame shown in hashed lines.

The term "area of interest of a subject" relates to a body part or portion of the subject, in which changes in macrovascular blood flow can be expected, e.g. due to an interventional procedure. The term macrovascular blood flow relates to blood flow in vessels that is visible for example in contrast injected X-ray images of the vessels.

The term "vascular region of interest" relates to a part or portion of the vascular structure of the subject, in which changes in the macrovascular blood flow are detected by the comparison of the image data relating to the different points in time.

The term "tissue portion of interest" relates to a part or portion of the tissue structure of the subject, in which changes in the macrovascular blood flow might most probably cause changes in the microvascular blood flow based on the feeding information for the respective identified vascular region of interest. The term microvascular blood flow relates to blood flow in the smaller parts of in particular the peripheral vascular structure, which parts are not or only hardly visible in contrast injected X-ray images of the vessels, but microvascular blood flow also relates to blood perfusion in tissue.

The term "surface portion" relates to a part or portion of the (outer, i.e. visible) surface of the respective tissue portion. The surface portion can also be referred to as surface area.

In an example, the surface portion is allocated on a representation of the surface of the subject, e.g. on an image of the surface of the subject. For example, an optical perfusion imaging is provided that captures a continuous stream of surface images and an indicator is overlaid to a presentation of the surface images to identify the surface portion.

In an example, the surface portion is allocated on the surface of the subject, e.g. directly on the surface of the subject, for example by an indicator projected onto the surface.

In an example, the allocated surface portion is provided as a preferred surface portion for optical surface perfusion measurements.

The term "data input" relates to providing or supplying data for data processing steps. The data input 12 can also be referred to as image data input. The data input can also be referred to as data supply, as image data supply, as image input, as input unit or simply as input. In an example, the image data input is data-connectable to an imaging source arrangement like an X-ray imaging system. In a further example, in addition or alternatively, the image data input is data-connectable to an optical camera providing the optical perfusion imaging of the subject. In an example, the data input is data-connected to an optical perfusion imaging source like a camera. In an example, the data input is data-connectable to a data storage having stored the optical perfusion image data.

The term "data processor" relates to a processor or part of a processor arrangement that is provided to conduct the computing steps using the data supplied by the data input. The data processor 14 can also be referred to as data processing arrangement, as processor unit or as processor. In an example, the data processor is data-connected to the data input and the output interface. In an example, the data processor is provided as vascular flow determination engine that detects and/or tracks macrovascular blood flow in the images. In an example, a contrast agent tracking over time is technically not required, e.g. if someone chooses a CCN or thresholding of the DSA for this purpose.

The term "output interface" relates to an interface for providing the processed or computed data for further purposes. The output interface 16 can also be referred to as output or output unit. In an example, the output interface is data-connectable to a display arrangement or display device. In another example, the output is data-connected to a display.

In an example, the first image data relates to a pre-interventional state of a vessel to be treated, and the second image data relates to a during- or post-interventional state. As an example, the intervention provides treatment of the vessel structure in order to improve blood flow at least in the vessel.

In an example, the surface portion is related to the determined at least one tissue portion of interest for microvascular perfusion.

In an example, the feeding information relates to the identified vascular region of interest. The feeding information may provide information on which perfused vessel (thus supplied with blood) supplies which tissue region.

The feeding information is based on a model of the semantic affiliation, i.e. a model defining which vessel supplies which tissue region. One approach is the identification of one or more regions in the model, for example in an angiosome model. Another approach is the assumption of a spatial proximity.

In an example, a supply vascular tree is provided for providing the feeding information.

Fig. 2 shows an example of a system 50 for monitoring peripheral perfusion. The system 50 comprises an X-ray imaging arrangement 52 for angiography of a region of interest of a subject. The system 50 also comprises an optical perfusion arrangement 54 for measurement of perfusion within a surface of the subject. The system 50 further comprises an example of the device 10 for monitoring peripheral perfusion according to one of the preceding examples. The X-ray imaging system 52 is configured to provide the angiographic image data. Further, the optical perfusion arrangement 54 is configured to conduct optical perfusion imaging of the allocated surface portion based on the surface portion identifier.

The X-ray imaging arrangement 52 can also be referred to as X-ray imaging system or as angiography imaging system. In an example, the angiography imaging system is provided as e.g. a C-arm structure. As an option, the angiography imaging system comprises a stereo configuration with respect to the optical imaging system presented by the optical perfusion arrangement, for example with known or measurable relative positioning/orientation, by using e.g. sensor hardware or by pose-estimation using the acquired images.

The optical perfusion arrangement 54 can also be referred to as optical perfusion imaging system, optical perfusion measurement arrangement or system, or as optical perfusion measurement device. In an example, the optical perfusion arrangement is provided e.g. as hyper- or multispectral iPPG with structured light.

Still further, a post-processing is provided that maps the measured signal, e.g. diffuse reflectance spectrum, to hemodynamic parameters, e.g. oxygen saturation.

In an example, the optical perfusion arrangement 54 is configured to conduct optical perfusion imaging at predetermined points in time.

In another example, the optical perfusion arrangement 54 is configured to conduct optical perfusion imaging in a continuous manner as an image stream.

In Fig. 2, the X-ray imaging arrangement 52 is provided comprising an X-ray source 56 and an X-ray detector 58 attached to a C-arm structure 60 movably mounted to a ceiling support 62. The device 10 for monitoring peripheral perfusion is provided integrated within a console arrangement that may comprise user interfaces such as displays, keyboards, control panels, touch pads, mouse and the like. The console is shown in the right foreground in Fig. 2. The X-ray imaging arrangement 52 is data-connected to the device 10 for monitoring peripheral perfusion as indicated by a first data-connection line 64. Further, a subject support 66 is shown as an adjustable table to provide support for a subject 68. Further equipment like a monitor arrangement 70 and lighting 72 are also indicated. The optical perfusion arrangement 54 is data-connected to the device 10 for monitoring peripheral perfusion as indicated by a second data-connection line 64.

As an option, shown in Fig. 2 with hashed lines, a spatial correspondence arrangement 76 is provided that is configured to provide relative spatial correspondence data of the X-ray imaging arrangement 52 and the optical perfusion arrangement 54. The spatial correspondence arrangement 76 is data-connected to the device 10 for monitoring peripheral perfusion as indicated by a third data-connection line 78.

The data-connections may be provided wire-bound or wireless.

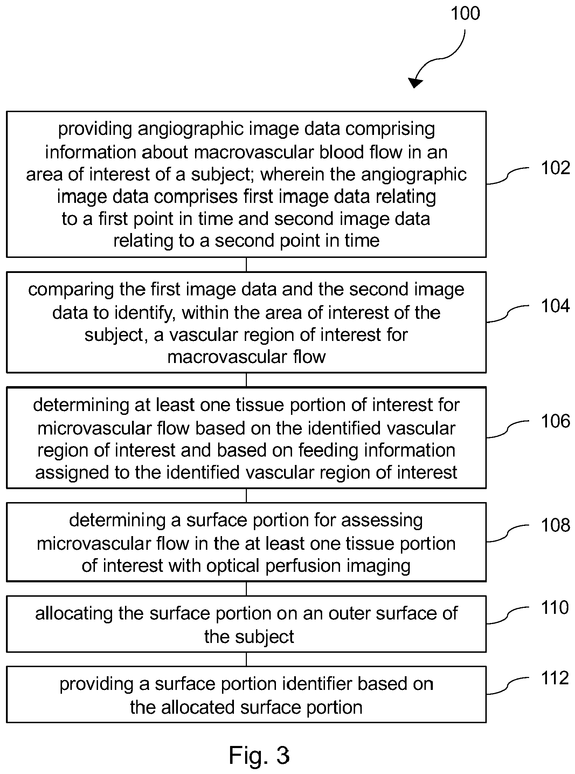

Fig. 3 shows steps of an example of a method 100 for peripheral perfusion imaging. The method 100 comprises the following steps:
- In a first step 102, angiographic image data comprising information about macrovascular blood flow in an area of interest of a subject is provided. The angiographic image data comprises first image data relating to a first point in time and second image data relating to a second point in time.
- In a second step 104, the first image data and the second image data are compared to identify, within the area of interest of the subject, a vascular region of interest for macrovascular flow.
- In a third step 106, at least one tissue portion of interest for microvascular perfusion is determined based on the identified vascular region of interest and based on feeding information assigned to the identified vascular region of interest.
- In a fourth step 108, a surface portion is determined for assessing microvascular perfusion in the at least one tissue portion of interest with optical perfusion imaging.
- In a fifth step 110, the surface portion is allocated on an outer surface of the subject.
- In a sixth step 112, a surface portion identifier is provided based on the allocated surface portion.

Referring back to Fig. 1 and Fig. 2, in an example, provided as an option, the data processor 14 is configured to generate a perfusion hypothesis based on the comparing of the first image data and the second image data and the identification of the vascular region of interest for macrovascular flow. The data processor 14 is configured to generate, as the identifier, instructions for optical perfusion imaging for at least one of the group of quantifying and validating of the perfusion hypothesis.

In an example, the generated perfusion hypothesis is used to identify and define instructions for the optical perfusion imaging for the quantifying and/or validating of the perfusion hypothesis.

In an example, the optical perfusion imaging is performed based on the instructions for the optical perfusion imaging.

For example, the instructions comprise the most relevant field-of-view for the optical measurement.

In an example, the quantifying comprises determining absolute values and relations of the measured optical perfusion image data.

In another example, alternatively or in addition, the validating comprises validating the perfusion hypothesis as expected form perfusion angiography against data from the measured optical perfusion image data.

In an example, the allocated surface portion is used for comparing expected surface perfusion differences with measured surface perfusion differences.

As an example, a mismatch of hypothesis and measured perfusion is detected via absolute values of the measurements.

As another example, a mismatch of hypothesis and measured perfusion is detected via differences in values or via pre- and post-images.

In an example, provided as an option, the data input 12 is configured to provide at least one of the group of locational information and semantic information for the vascular structure of at least one of the group of the first image data and the second image data. Further, the data processor 14 is configured to determine the at least one tissue portion of interest for microvascular perfusion using the semantic information. As an additional or alternative option, the data processor 14 is configured to allocate the surface portion using the locational information.

In an example, the locational information is based on segmenting vessels in the angiographic image data. The segmenting can be provided manually by the user or by a segmentation algorithm.

The term locational information relates to data relating to a relative position of the vascular structure. The locational information thus provides the spatial context.

The term semantic information relates to data relating to an anatomical context of the particular part of the vascular structure, such as which sub-branches or which tissue regions are commonly supplied by the part of the vascular structure. The semantic information thus provides the functional context.

In an example, 2D angio images are provided with labels for vessel segments, thus providing 3D information over the generic model, for example. This may be provided in order to assess if vascular regions are supplied with blood flow.

In an example, provided as an option, the data processor 14 is configured to register the angiographic image data and the subject to a common spatial reference frame. Further, the data processor 14 is configured to allocate the surface portion based on the common spatial reference frame.

In an example, provided as an option, the angiographic image data comprises spatial anatomical information comprising a plurality of vessel segments. For the identification of the vascular region of interest for macrovascular flow, the data processor 14 is configured to identify at least one of the plurality of vessel segments. The feeding information comprises segments of tissue portions that are assigned to vessel segments of the plurality of vessel segments as being supplied by these vessel segments.

In an example, the angiographic image data comprises a number of angiographic projection images of the subject from at least two different directions such that the angiographic image data comprises is providing spatial information of the subject.

In an example, provided as an option, the feeding information comprises a feeding territory model. The data processor 14 is configured to spatially register the angiographic image data to the feeding territory model. The data processor 14 is also configured to register the feeding territory model to the subject.

In an example, an anatomical model is provided that is linked to the feeding territory model. The angiographic image data is registered to the anatomical model. In an option, also the optical perfusion image data is registered to the anatomical model.

In an example, provided as an option, the area of interest of the subject comprises the lower limbs. The at least one tissue portion of interest for microvascular perfusion comprises at least one foot region of the subject.

In an example, provided as an option, the output interface 16 is configured to provide optical perfusion image data. The optical perfusion image data comprises at least one 2D optical perfusion image of the surface of the subject. The data processor 14 is configured to generate an indicator for the allocated surface portion. The data processor 14 is configured to overlay the indicator to the at least one 2D optical perfusion image to provide a location augmented optical perfusion image. The output interface 16 is configured to provide the location augmented optical perfusion image for a perfusion assessment in the image portion defined by the indicator.

The augmented image provides information where measurements are relevant in an intuitive manner.

In an example, provided as an option, for overlaying the indicator, the data processor 14 is configured to register the at least one 2D optical perfusion image with the angiographic image data.

In an example, for registering the at least one 2D optical perfusion image and the angiographic image data, a spatial relation between the two imaging modalities is determined.

In an example, the spatial relation is provided based on the angiographic image data.

In another example, a tracking arrangement, e.g. like the spatial correspondence arrangement 76, is provided that tracks a spatial relation of the X-ray imaging system 52 that provides the angiographic image data and an optical perfusion imaging system that provides the optical perfusion image data.

In an example, provided as an option, the data input 12 is configured to provide location data representing a relative spatial arrangement of an angiography imager when acquiring the angiographic image data and an optical measurement imager for acquiring the optical perfusion imaging. Further, the data processor 14 is configured to generate position adjustment instructions to arrange the optical measurement imager for providing measurement of the determined surface portion of the subject.

The term angiography imager relates to an imaging device or system capable of providing the angiographic image data.

The term optical measurement imager relates to an imaging device or system capable of providing the optical perfusion image data.

In an example, an angiography driven field-of-view selection for optical perfusion measurement is provided. If the optical perfusion measurement can only be applied to a limited field-of-view, the angiography imaging can be used for selecting the most relevant area to be measured in order to speed-up the clinical workflow. For this purpose, the angiography is acquired first. Having identified e.g. the clinically most relevant sole region in the angiography, e.g. a region with perfusion deficits in the depth where an optical measurement of the skin oxygenation is of clinical interest, the measured/known relative pose of the angiography and the optical measurement device is used to move the latter in space such that this most relevant sole region is fully covered. Such an angiography-driven field-of-view selection is provided, for example, if there are no visible signs of perfusion deficits on the skin surface, e.g. only slowly healing wounds.

In an example, the spatial correspondence arrangement 76 establishes the spatial correspondence between the two imaging modalities and transfers location information and hypotheses about the perfusion status from angiography to the optical surface domain for an advanced analysis of perfusion properties.

The spatial correspondence between the two imaging modalities is needed for registering both image domains or image spheres with each other.

In an example, angio images are provided together with information about the vascular types or segments. This allows a registration with the optical perfusion images.
In another example, the angio images are provided without spatial information. A model is provided in order to assess spatial data such that the registration with the optical perfusion images is possible.

In an example, both optical perfusion measurements, e.g. hyper/multispectral iPPG with structured light, and angiography sequences are acquired from approximately orthogonal views. If e.g. cuff or other stimuli are applied, measurements are synchronized. In an example, in order to establish approximate spatial correspondences between the modalities, the imaging geometry, i.e. relative positioning of modalities and e.g. a foot, must be known or reconstructed: While a hardware-wise fixation, e.g. mounting the optical imaging system on the C-arm, might not be feasible, e.g. due to geometric constraints, the relative position and rotation of both imaging devices may be measured via sensors, e.g. (depth-) camera, Wi-Fi, radar. In one example, at least one ceiling mounted camera senses the position of reflective or active marker constellations attached to the gantry and the optical imaging system in cathlab coordinates. In another example, at least two angiography sequences are acquired from different views. As an alternative to sensors, pose-estimation algorithms are be applied to both the angiography and optically acquired images: For the angiography, a neural-network based matching of an articulated foot-model to the X-ray image is used. Utilizing the known change in the C-arm positioning, e.g. encoded in the DICOM metadata, the accuracy of the pose-estimation of angiography based on the acquired images can be enhanced. In an example, a pose of the optical imaging system is estimated using the acquired RGB or grey-level signal. This may be easily accomplished by using standard triangulation approaches with a second camera or projection device, e.g. light spatially structured in the plane orthogonal to its propagation direction.

In an example of a clinical workflow, DSA sequences are acquired prior to and after the lesion has been treated during an actual intervention. Both sequences, i.e. pre and post, show the macrovascular tree, which is perfused, i.e. contrast filled, at the time of imaging. The impact of the intervention on macrovascular perfusion can be assessed by comparing the two vessel trees. A perfused part of the vessel tree, and therefore also a change in perfusion, can be defined in one of the following ways:
Binary fashion: According to a certain intensity threshold on the contrast density, a vessel or part of the vessel is either perfused or not. Interventional impact is identified by vessel segments that are filled by a contrast density above the threshold after the intervention, but not before. The analysis can be done after reducing the DSA sequence to its MIP and segmenting the macrovascular tree. In an example, a segmentation mask is used to eliminate image parts which do not belong to the macrovascular tree before applying the threshold.
Time-density curve (TDC) parameter change: A softer criterium is applied, which analyses the contrast density curve of each pixel in the macrovasculature and extracts parameters from that curve, e.g. peak density, time-to-peak, plateau width and the like. In addition to newly appearing vessel segments, significant changes in one of the TDC parameters also qualify a vessel segment to be highlighted as impacted by the intervention.

In an example, the way the change in perfusion is measured provides an influence on how the (re-)prefusion hypothesis for the optical part is defined. While the thresholding approach above indicates that a perfusion change is expected optically which is maybe above some inter-repeated-measurement variability, the TDC approach is capable of also indicating the extent of expected surface perfusion change.

As an example, the analysis of a foot angiogram results in three outputs: the pre-interventionally perfused macrovasculature, the post-interventionally perfused macrovasculature and the part of the vessel tree that was affected by the intervention.

In an example, both angiographic X-ray projections (pre and post) are recorded from very similar angles as required by a stereo-imaging setup, their DSA-based MIPs (masked by the macrovascular segmentation) are warped onto each other to define the differences in the vascular trees. The warping, e.g. via thin plate splines or optical flow, matches coinciding parts of the vascular tree and lets the differing parts stand out.

In an example, locational and semantic information to these outputs is provided manually (by the user) or automatically, assigning vessel labels to vascular segments of the vessel tree. Vascular segments are defined and bordered by branching points/bifurcations and are assigned a vessel label, such as ATA1, ATA2, ..., PTA1, PTA2, when identifiable from a reference anatomical atlas.

An automatic extraction of this information could start as an iterative approach seeded at the inlet vessels in the ankle region. From there the tracking travels down the vascular tree detecting and branching at encountered bifurcations.

Further, the outputs can be connected to a 3D reference model of the foot where they are linked to their corresponding feeding territories in the tissue. Here, an exact registration of the X-ray projected foot pose to the 3D mode is not necessary, because semantic information has been found in the 2D vessel tree, which can be used to identify correspondences to supply regions in the 3D model to which the optical scan is registered.

This can be done in several ways: By exploiting the vessel label and the angiosome model, a general population-wide model which indicates which parts of the foot are perfused by which vessel (ATA, PTA, peroneal, calcaneal artery ...); or by using an average vascular tree as part of the reference model, that was built in a data-driven manner from many contrasted 3D images of feet (CTA, MRA data) and that contains the vascular segment labels annotated in the DSA image. As an approximation, it is assumed (and computed via nearest neighbor search) that tissue regions closest to a particular vessel are also perfused by this vessel. Regions in the 3D model for which the perfusing vessel segment (present in the average reference tree in the foot model) is not observed/perfused in the angiogram, are labelled as not perfused or if a pre/post-interventional change was detected are labelled as impacted by the intervention; or diseased patients may exhibit a very individual vascular tree especially at the smaller caliber parts of the tree. This also includes collaterals and changes in the vasculature that have built up over time to compensate for the progressing disease. These aspects are less likely to be covered by the angiosome model or a standard reference vascular tree (if it does not come from a patient-specific 3D image). In this case, the connectivity (branching point locations) to the bigger caliber vessels (included in the angiosome model) can be exploited to estimate which tissue parts may be affected by a perfusion change in this individual part of the vascular tree or angiosome. Depending on the connectivity, this could give rise to a further narrowing down the affected sub-parts within the coarser angiosome parcellation.

By this procedure, it can be approximated which parts of the surface tissue are perfused to which extent and how it has been changing during the intervention.

Further, the hyper/multispectral iPPG surface scan can also be mapped to the reference foot model. By having both pieces of information (affected and relevant perfusion territory information from angiography & iPPG surface perfusion parameter map), the information can be overlaid on each other to evaluate the correlation between the interventional impact (macrovascular vessel tree information), its hypothesized impact on the perfusion change in the feeding territory (the mapping procedure in the foot model) as well as the actual impact on the surface tissue as observed by iPPG.

Expectations that the interventionalist has based on his or her treatment, can therefore be automatically compared with the real effect in the target region. The information can be automatically reported, and disagreements can be highlighted to the clinician. Going through this procedure several times, for example after the clinician has possibly extended the treatment, e.g. additional ballooning, opening another vessel etc., results in a documentation of incremental improvements. These provide insights into how changes in the surface tissue supply are related to macrovascular changes caused by the interventional effort, which can be used for a prognosis of what effects are still likely to be achieved via the intervention.

In an example of the method, a perfusion hypothesis is generated based on the comparing of the first image data and the second image data and the identification of the vascular region of interest for macrovascular flow. Further, as the identifier, instructions for optical perfusion imaging are generated for at least one of the group of quantifying and validating of the perfusion hypothesis.

In an example of the method, locational information and semantic information are provided for the vascular structure of at least one of the group of the first image data and the second image data. The semantic information is provided for the determining of the at least one tissue portion of interest for microvascular perfusion. The locational information is provided for the allocating of the surface portion.

In an example of the method, the angiographic image data and the subject are registered to a common spatial reference frame. The allocating of the surface portion comprises allocating a surface portion of the subject based on the common spatial reference frame.

In an example of the method, the angiographic image data comprises spatial anatomical information comprising a plurality of vessel segments. The identification of the vascular region of interest for macrovascular flow comprises identifying at least one of the plurality of vessel segments. The feeding information comprises segments of tissue portions that are assigned to vessel segments of the plurality of vessel segments as being supplied by these vessel segments.

In an example of the method, the feeding information comprises a feeding territory model. The angiographic image data is spatially registered to the feeding territory model. The feeding territory model is registered to the subject.

In an example of the method, the area of interest of the subject comprises the lower limbs. The at least one tissue portion of interest for microvascular perfusion comprises at least one foot region of the subject.

In an example of the method, optical perfusion image data is provided. The optical perfusion image data comprises at least one 2D optical perfusion image of the surface of the subject. An indicator is generated for the allocated surface portion; and the indicator is overlaid to the at least one 2D optical perfusion image to provide a location augmented optical perfusion image. The location augmented optical perfusion image is provided for a perfusion assessment in the image portion defined by the indicator.

In an example of the method, for overlaying the indicator, the at least one 2D optical perfusion image is registered with the angiographic image data.

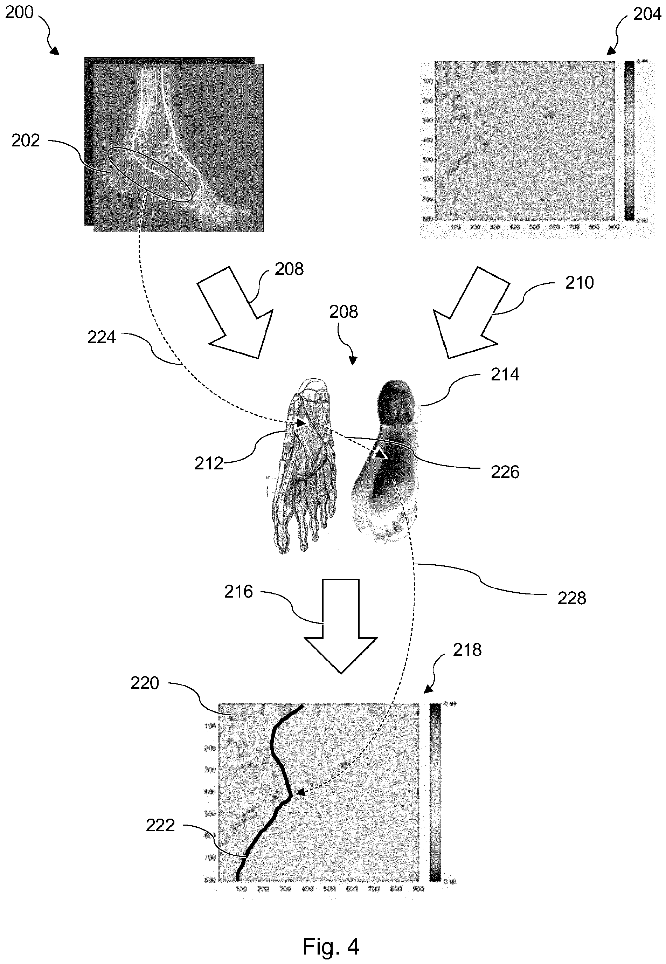

Fig. 4 illustrates an example of a workflow for peripheral perfusion imaging. Fig. 4 shows a schematic illustration of information flow for transferring expectations arising from macrovascular aspects as observed in the angiogram to the optically scanned skin surface area. The latter is assumed to be partially fed by the relevant macrovascular parts. The transfer is accomplished by using a vascular model that carries or approximates the information of which tissue is fed by which artery. Depending on the level of detail in the vascular tree, the feeding zones can be organized in a refinement hierarchy becoming finer with decreasing vessel calibers.

As indicated in Fig. 4, at least one pre interventional angiogram and at least one post interventional angiogram are provided, indicated with reference numeral 200. As an example, a perfusion dysfunction in lateral plantar artery is detected by comparing the pre and post angiograms, as indicated with reference numeral 202. Further, an optical perfusion image 204 is provided. In a first registration part 206, the pre/post angiograms 200 are registered to a vasculature/feeding territory model 208, e.g. an angiosome model. In a second registration part 210, the optical perfusion image 204 is registered to the vasculature/feeding territory model 208, e.g. the angiosome model. In Fig. 4, an anatomical foot model 212 is shown, together with a territory model 214. In a computational procedure, a surface portion for assessing microvascular perfusion in the at least one tissue portion of interest with optical perfusion imaging is determined. In an augmentation 216, data for displaying an augmented optical perfusion image 218 is provided which is then displayed showing optical perfusion image content 220 together with a surface portion identifier 222. Hence, by detecting the area of interest in the angiographic image data, this location is transferred to the foot model 212, as indicated by first hashed arrow 224, to determine the tissue portion of interest. This is then transferred to the territory model 214, as indicated by second hashed arrow 226, to determine the surface portion. From here, a further transformation is provided by allocating the surface portion on an outer surface of the subject, as indicated by third hashed arrow 228, to be able to present the surface portion identifier 222.

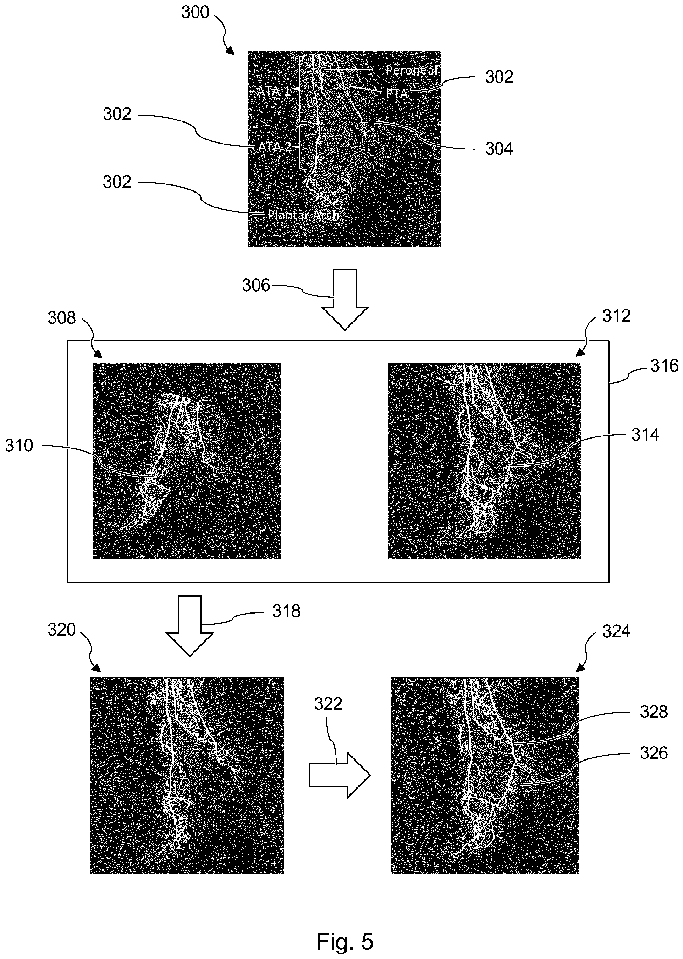

Fig. 5 illustrates a further example of a workflow for peripheral perfusion imaging. Fig. 5 shows extracting macrovascular information from pre- and post-interventional images. The macrovasculature is first segmented from the DSA MIP image and semantic labels are assigned to segments of the vessel tree. This procedure is done for the pre- and post-interventional image (top row). Slightly differing projection angles or foot poses between the two images are compensated by geometrically warping the coordinates of one into the other image (e.g. via thin-plate splines (TPS)). Using this match, the diff between the two vessel trees can be determined (bottom right in green). Finally, there are three pieces of information available for highlighting blood supply regions in the surface scan: pre-interventional macrovasculature, post-interventional macrovasculature, difference in macrovasculature between pre and post.

As indicated in Fig. 5, image data 300 of a digital subtraction angiography with and maximum intensity projection (DSA MIP) is provided. Semantic labels 302 are indicated for a macrovascular structure 304. A segmentation 306 results in a first macrovascular image 308 with a macrovascular structure 310, which relates to a pre interventional state, and a second macrovascular image 312 with a macrovascular structure 314, which relates to a post interventional state. A frame 316 indicates the option of a common data processing. Further, a warping procedure 318 results in a warped image 320. A difference is provided in a further procedure 322 resulting in a difference-image 324 in which differing parts 326 of the macrovascular structure 328 of the subject are highlighted.

The term "subject" may also be referred to as individual. The "subject" may further also be referred to as patient, although it is noted that this term does not indicate whether any illness or disease is actually present with the subject.

In an example, a computer program is provided that enables a processor to carry out the method of the examples above.

In an example, a computer program or program element for controlling an apparatus according to one of the examples above is provided, which program or program element, when being executed by a processing unit, is adapted to perform the method steps of one of the method examples above, on an appropriate system.

In an example, a computer readable medium is having stored the program element of the previous example.

The computer program element might therefore be stored on a computer unit or be distributed over more than one computer units, which might also be part of an embodiment of the present invention. This computing unit may be adapted to perform or induce a performing of the steps of the method described above. Moreover, it may be adapted to operate the components of the above described apparatus. The computing unit can be adapted to operate automatically and/or to execute the orders of a user. A computer program may be loaded into a working memory of a data processor. The data processor may thus be equipped to carry out the method of the invention.

Aspects of the invention may be implemented in a computer program product, which may be a collection of computer program instructions stored on a computer readable storage device which may be executed by a computer. The instructions of the present invention may be in any interpretable or executable code mechanism, including but not limited to scripts, interpretable programs, dynamic link libraries (DLLs) or Java classes. The instructions can be provided as complete executable programs, partial executable programs, as modifications to existing programs (e.g. updates) or extensions for existing programs (e.g. plugins). Moreover, parts of the processing of the present invention may be distributed over multiple computers or processors.

As discussed above, the processing unit, for instance a controller implements the control method. The controller can be implemented in numerous ways, with software and/or hardware, to perform the various functions required. A processor is one example of a controller which employs one or more microprocessors that may be programmed using software (e.g., microcode) to perform the required functions. A controller may however be implemented with or without employing a processor, and also may be implemented as a combination of dedicated hardware to perform some functions and a processor (e.g., one or more programmed microprocessors and associated circuitry) to perform other functions.

Examples of controller components that may be employed in various embodiments of the present disclosure include, but are not limited to, conventional microprocessors, application specific integrated circuits (ASICs), and field-programmable gate arrays (FPGAs).

This exemplary embodiment of the invention covers both, a computer program that right from the beginning uses the invention and a computer program that by means of an up-date turns an existing program into a program that uses the invention.

Further on, the computer program element might be able to provide all necessary steps to fulfil the procedure of an exemplary embodiment of the method as described above.

According to a further exemplary embodiment of the present invention, a computer readable medium, such as a CD-ROM, is presented wherein the computer readable medium has a computer program element stored on it which computer program element is described by the preceding section. A computer program may be stored and/or distributed on a suitable medium, such as an optical storage medium or a solid-state medium supplied together with or as part of other hardware, but may also be distributed in other forms, such as via the internet or other wired or wireless telecommunication systems.

However, the computer program may also be presented over a network like the World Wide Web and can be downloaded into the working memory of a data processor from such a network. According to a further exemplary embodiment of the present invention, a medium for making a computer program element available for downloading is provided, which computer program element is arranged to perform a method according to one of the previously described embodiments of the invention.

It has to be noted that embodiments of the invention are described with reference to different subject matters. In particular, some embodiments are described with reference to method type claims whereas other embodiments are described with reference to the device type claims. However, a person skilled in the art will gather from the above and the following description that, unless otherwise notified, in addition to any combination of features belonging to one type of subject matter also any combination between features relating to different subject matters is considered to be disclosed with this application. However, all features can be combined providing synergetic effects that are more than the simple summation of the features.

While the invention has been illustrated and described in detail in the drawings and foregoing description, such illustration and description are to be considered illustrative or exemplary and not restrictive. The invention is not limited to the disclosed embodiments. Other variations to the disclosed embodiments can be understood and effected by those skilled in the art in practicing a claimed invention, from a study of the drawings, the disclosure, and the dependent claims.

In the claims, the word "comprising" does not exclude other elements or steps, and the indefinite article "a" or "an" does not exclude a plurality. A single processor or other unit may fulfil the functions of several items re-cited in the claims. The mere fact that certain measures are re-cited in mutually different dependent claims does not indicate that a combination of these measures cannot be used to advantage. Any reference signs in the claims should not be construed as limiting the scope.

## Claims

1. A device (10) for monitoring peripheral perfusion, comprising:
- a data input (12);
- a data processor (14); and
- an output interface (16);
wherein the data input is configured to provide angiographic image data comprising information about macrovascular blood flow in an area of interest of a subject; wherein the angiographic image data comprises first image data relating to a first point in time, and second image data relating to a second point in time;
wherein the data processor is configured to compare the first image data and the second image data to identify, within the area of interest of the subject, a vascular region of interest for macrovascular flow; to determine at least one tissue portion of interest for microvascular perfusion based on the identified vascular region of interest and based on feeding information assigned to the identified vascular region of interest; to determine a surface portion for assessing microvascular perfusion in the at least one tissue portion of interest with optical perfusion imaging; and to allocate the surface portion on an outer surface of the subject; and
wherein the output interface is configured to provide a surface portion identifier based on the allocated surface portion.

2. Device according to claim 1, wherein the data processor is configured to generate a perfusion hypothesis based on the comparing of the first image data and the second image data and the identification of the vascular region of interest for macrovascular flow; and
wherein the data processor is configured to generate, as the identifier, instructions for optical perfusion imaging for at least one of the group of: quantifying and validating of the perfusion hypothesis.

3. Device according to claim 1 or 2, wherein the data input is configured to provide at least one of the group of locational information and semantic information for the vascular structure of at least one of the group of the first image data and the second image data;
wherein the data processor is configured to determine the at least one tissue portion of interest for microvascular perfusion using the semantic information; and/or
wherein the data processor is configured to allocate the surface portion using the locational information.

4. Device according to claim 1, 2 or 3, wherein the data processor is configured to register the angiographic image data and the subject to a common spatial reference frame; and
wherein the data processor is configured to allocate the surface portion based on the common spatial reference frame.

5. Device according to one of the preceding claims, wherein the angiographic image data comprises spatial anatomical information comprising a plurality of vessel segments;
wherein, for the identification of the vascular region of interest for macrovascular flow, the data processor is configured to identify at least one of the plurality of vessel segments; and
wherein the feeding information comprises segments of tissue portions that are assigned to vessel segments of the plurality of vessel segments as being supplied by these vessel segments.

6. Device according to one of the preceding claims, wherein the feeding information comprises a feeding territory model;
wherein the data processor is configured to spatially register the angiographic image data to the feeding territory model; and
wherein the data processor is configured to register the feeding territory model to the subject.

7. Device according to one of the preceding claims, wherein the area of interest of the subject comprises the lower limbs; and
wherein the at least one tissue portion of interest for microvascular perfusion comprises at least one foot region of the subject.

8. Device according to one of the preceding claims, wherein the output interface is configured to provide optical perfusion image data; wherein the optical perfusion image data comprises at least one 2D optical perfusion image of the surface of the subject;
wherein the data processor is configured to generate an indicator for the allocated surface portion;
wherein the data processor is configured to overlay the indicator to the at least one 2D optical perfusion image to provide a location augmented optical perfusion image; and
wherein the output interface is configured to provide the location augmented optical perfusion image for a perfusion assessment in the image portion defined by the indicator.

9. Device according to one of the preceding claims, wherein, for overlaying the indicator, the data processor is configured to register the at least one 2D optical perfusion image with the angiographic image data.

10. Device according to one of the preceding claims, wherein the data input is configured to provide location data representing a relative spatial arrangement of an angiography imager when acquiring the angiographic image data and an optical measurement imager for acquiring the optical perfusion imaging; and
wherein the data processor is configured to generate position adjustment instructions to arrange the optical measurement imager for providing measurement of the determined surface portion of the subject.

11. A system (50) for monitoring peripheral perfusion, comprising:
- an X-ray imaging arrangement (52) for angiography of a region of interest of a subject;
- an optical perfusion arrangement (54) for measurement of perfusion within a surface of the subject; and
- a device for monitoring peripheral perfusion (10) according to one of the preceding claims;
wherein the X-ray imaging system is configured to provide the angiographic image data; and
wherein the optical perfusion arrangement is configured to conduct optical perfusion imaging of the allocated surface portion based on the surface portion identifier.

12. System according to claim 11, wherein a spatial correspondence arrangement (76) is provided that is configured to provide relative spatial correspondence data of the X-ray imaging arrangement and the optical perfusion arrangement.

13. A method (100) for peripheral perfusion imaging, the method comprising the following steps:
- providing (102) angiographic image data comprising information about macrovascular blood flow in an area of interest of a subject; wherein the angiographic image data comprises first image data relating to a first point in time and second image data relating to a second point in time;
- comparing (104) the first image data and the second image data to identify, within the area of interest of the subject, a vascular region of interest for macrovascular flow;
- determining (106) at least one tissue portion of interest for microvascular perfusion based on the identified vascular region of interest and based on feeding information assigned to the identified vascular region of interest;
- determining (108) a surface portion for assessing microvascular perfusion in the at least one tissue portion of interest with optical perfusion imaging;
- allocating (110) the surface portion on an outer surface of the subject; and
- providing (112) a surface portion identifier based on the allocated surface portion.

14. A computer program enabling a processor to carry out the method of claim 13.

15. A computer readable medium having stored the program element of claim 14.
